(19) Europäisches Patentamt
European Patent Office
Office européen des brevets

(11) Publication number: 0 174 155 A2

# (12) EUROPEAN PATENT APPLICATION

(21) Application number: 85306098.6

(22) Date of filing: 28.08.85

(51) Int. Cl.[4]: C 12 N 1/38
C 07 D 471/04

(30) Priority: 30.08.84 JP 179344/84

(43) Date of publication of application:
12.03.86 Bulletin 86/11

(84) Designated Contracting States:
DE FR GB

(71) Applicant: Ube Industries, Ltd.
12-32, Nishihonmachi 1-chome
Ube-shi, Yamaguchi-ken(JP)

(72) Inventor: Ameyama, Minoru
44-1, Ouchimihori
Yamaguchi-shi Yamaguchi(JP)

(72) Inventor: Adachi, Osao
2034, Shimohirano
Miyano Yamaguchi-shi Yamaguchi(JP)

(74) Representative: Arthur, Bryan Edward et al,
Withers & Rogers 4 Dyer's Buildings Holborn
London EC1N 2JT(GB)

## (54) Cell growth promoting agent and process for promoting cell growth.

(57) A cell growth promoting agent for microorganism, plant and animal cells, comprising 4,5-dihydro-4, 5-dioxo-1H-pyrrolo [2,3-f]quinoline-2,7,9-tricarboxylic acid (PQQ); and a process for promoting the growth of microorganism, plant or animal cells, comprising culturing the cells in a culture medium containing an effective amount of PQQ.

EP 0 174 155 A2

Croydon Printing Company Ltd.

# CELL GROWTH PROMOTING AGENT AND PROCESS FOR PROMOTING CELL GROWTH

## BACKGROUND OF THE INVENTION

### 1. Field of the Invention

The present invention relates to a cell growth promoting agent comprising 4,5-dihydro-4,5-dioxo-1H-pyrrolo[2,3-f]quinoline-2,7,9-tricarboxylic acid (hereinafter abbreviated as PQQ) and a process for promoting cell growth using PQQ.

### 2. Description of the Related Art

Various kinds of useful compounds are industrially produced using microorganisms. Such compounds include alcohols such as ethanol and buthanol, oxidized sugar alcohols such as L-sorbose and dibydroxyacetone, organic acids such as acetic acid, citric acid, lactic acid, and gluconic acid, amino acids such as glutamic acid, lysine, and valine, nucleic acids and related compounds such as adenine, guanine, cytosine, uracil, and inosinic acid, vaccines for such as hepatitis B virus and other pathogenic viruses and bacteria, antibiotics such as penicillin, streptomycin, and erythromycin, and enzymes such as α-amylase, glucoamylase, protease, invertase, lipase, and pectinase. Recently, further useful compounds such as insulin, interferons, interleukins, growth hormones, and antibodies can be produced by gene recombinant techniques and cell fusion techniques using microorganisms, animal or plant cells: some of which compounds are now industrially produced. When such useful compounds are produced using microorganisms, plant cells or animal cells, it is very important to shorten the culturing time to enhance the efficiency of the production of the aimed compound.

At present, to promote the growth of cells and to shorten the culturing time, natural products and

extracts thereof, such as yeast extract, meat extract, corn steep liquor, serum, and rumen juice are used. However, such cell growth promoting products are usually a mixture of many different ingredients and the majority of such ingredients are ineffective for promoting cell growth and shortening the culturing time. Therefore, such growth promoting products are used in rather large amounts, to obtain sufficient promotion of the cell growth and shortening of the culturing time, and this in turn makes purification of the aimed compound produced during the culturing difficult. Moreover, active ingredients included in such conventional growth promoting products are, due to their natural origin, usually difficult to identify, and their composition often varies depending on lots used for their preparation. Therefore, to determine an optimum amount of the growth promoting products to be added, it is necessary to carry out tedious and time-consuming tests, lot by lot, of the products.

Such drawbacks in the conventional cell growth promoting products lessen the advantages thereof provided from their cell growth promoting activity. Therefor, a new cell growth promoting agent is desired, in which a compound effective at a low level of the concentration is identified and thus avoids adverse affects on the purification of an aimed product.

SUMMARY OF THE INVENTION

Accordingly, an object of the present invention is to provide a new cell growth promoting agent for microorganisms, plant and animal cells, comprising 4,5-dihydro-4,5-dioxo-1H-pyrrolo[2,3-f]quinoline-2,7,9-tricarboxylic acid (PQQ).

Another object of the present invention is to provide a process for promoting growth of microorganisms, plant or animal cells, comprising culturing the cells in a culture medium containing an effective amount of 4,5-dihydro-4,5-dioxo-1H-pyrrolo[2,3-f]quinoline-2,7,9-tricarboxylic acid (PQQ).

BRIEF DESCRIPTION OF THE DRAWINGS

Figure 1 is a graph showing the relationship between PQQ concentration in a culture medium and extent of growth when Gluconobacter industrius IFO 3260 was cultured according to Example 1;

Fig. 2 is a graph showing the difference in cell growth in the presence and in the absence of PQQ when Acetobacter aceti IFO 3284 was cultured according to Example 2;

Fig. 3 is a graph showing the differences in cell growth, in 5-keto-D-gluconic acid production, and in gluconate dehydrogenase activity in the presence and in the absence of PQQ when Gluconobacter oxydans IFO 3287 was cultured according to Example 3;

Fig. 4 is a graph showing the differences in acetic acid production, in alcohol dehydrogenase activity, and in aldehyde dehydrogenase activity in the presence and in the absence of PQQ when Acetobacter aceti IFO 3284 was cultured according to Example 4;

Fig. 5 is a graph showing the difference in cell growth in the presence and in the absence of PQQ when Acetobacter rancens IFO 3297 was cultured according to Example 5;

Fig. 6 is a graph showing the difference in cell growth in the presence and in the absence of PQQ when Saccharomyces sake (Syn. Saccharomyces cerevisiae, Brewing Society of Japan, No. 7 yeast) was cultured according to Example 6;

Fig. 7 is a graph showing the difference in cell growth in the presence and in the absence of PQQ when Schizosaccharomyces pombe IFO 0346 was cultured according to Example 7;

Fig. 8 is a graph showing cell growth rates and total growths dependent on PQQ concentrations when Pseudomonas sp. VM15C was cultured according to Example 8;

Fig. 9 is a graph showing the relationship between an amount of cell mass and PQQ concentration in a medium when tobacco cells were cultured according to Example 9; and,

Fig. 10 is a graph showing the difference in cell number in the presence and in the absence of PQQ when Vero cells (African green monkey established cells) were cultured according to Example 11.

DESCRIPTION OF THE PREFERRED EMBODIMENT

According to the present invention, an active ingredient of a cell growth promoting agent is 4,5-dihydro-4,5-dioxo-1H-pyrrolo[2,3-f]quinoline-2,7,9-tricarboxylic acid (PQQ) having the following formula:

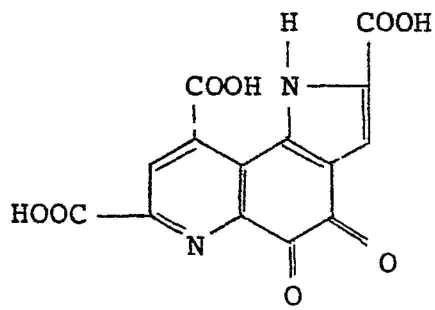

or salts thereof.

The PQQ was first found as a coenzyme for methanol dehydrogenase found in methanol-utilizing microorganisms in 1979. Although PQQ was expected to serve as a co-factor in various kinds of redox enzyme systems, it was not thought to show cell growth promoting activity. Surprisingly, the present inventor has now discovered that PQQ promotes the growth of cells, including microorganisms, plant and animal cells.

PQQ may be produced by chemical synthesis according to, for example, a process described by Corey et.al., Journal of American Chemical Society, 103, 5599 (1981), or by fermentation according to, for example, a process described in Japanese Patent Application No. 57-220,328

(Japanese Unexamined Patent Publication No. 59-113896) by the present applicant.

PQQ promotes the growth of various kinds of cells including microorganisms, plant and animal cells. The microorganisms include bacteria, yeast, fungi, and actinomycetes. The bacteria include, for example, genus Gluconobacter, such as Gluconobacter industrius, Gluconobacter oxydans, genus Acetobacter, such as Acetobacter aceti, Acetobacter rancens, genus Pseudomonas, genus Corynebacterium, genus Escherichia, and genus Baccillus. The yeasts include, for example, genus Saccharomyces, such as Saccharomyces cerevisiae, genus Schizosaccharomyces such as Schizosaccharomyces pombe, genus Torula, genus Candida. The actinomycetes include genus Streptomyces, and genus Nocardia. The fungi, for example, include genus Aspergillus, genus Penicillium. The term "Plant cells" as used herein is intended to include callus and cell lines derived from various kinds of plants, which may produce useful substances, for example, tobacco cells, Lithospermum cells, and Panax cells. The term "Animal cells" as used herein should be understood to include cell lines and cell strains derived from various kinds of organs, and tissues or cells of animals, which may produce useful substances. The cell lines especially include hybridoma cell lines derived from tumor cell and antibody-producing B lymphocyte. The growth of established experimental cell strains such as HeLa cell is also promoted by PQQ.

As a result of the promotion of cell growth by PQQ, the time required to reach the maximum cell concentration is shortened. Moreover, when a useful product is produced by the cultured cells, the time required to reach the maximum yield amount of the product is usually shortened, and surprisingly, the maximum yield amount of the product produced in the presence of PQQ is in many cases substantially higher than that in the absence of PQQ. That is, in addition to promotion of cell growth, PQQ

also promotes the production of an aimed product from a point of view of both production rate and yield amount, thus providing practical and economical advantages in the industrial production of useful products.

The effective concentration of PQQ in a culture medium is usually 0.01 ng/ml to 50 μg/ml, preferably 0.1 ng/ml to 1 μg/ml. The effective concentration is much lower than that of the conventional cell growth promoting materials, such as yeast extract and meat extracts. Therefore the addition of PQQ to promote cell growth and the production of an aimed product does not adversely affect the purification of the aimed product, which provides additional advantages.

A cell growth promoting agent according to the present invention may be a PQQ preparation per se. Moreover, the present agent may comprise PQQ and one or more appropriate additives. The additives are preferably the usual ingredients used for a culture medium, such as a stock solution of inorganic salts, a vitamin mixture, and the like.

PQQ can be in the form of a salt such as sodium salt or potassium salt.

The present invention also relates to a process for promoting cell growth in the culturing of microorganisms, plant or animal cells, which comprises adding an effective amount of PQQ to a culture medium to promote the growth of cells. The effective addition amount is as described above. PQQ may be added as a PQQ preparation per se, or as a preparation comprising PQQ and other additives: these are conveniently added to a culture medium prior to the inoculation of cells, or they can be added stepwise or continuously during the culturing of the cells. The culture conditions including composition of a culture medium, pH value of the medium, and temperature are the same as for a conventional process for culturing cells, which may vary according to the type of cells to be cultured, or other factors.

EXAMPLES

The present invention will now be further illustrated by, but is by no means limited to, the following examples.

Example 1

3 ml of a culture medium containing glycerin 4 g/l, monosodium glutamate 6 g/l, $KH_2PO_4$ 500 mg/l, $K_2HPO_4$ 500 mg/l, $MgSO_4 \cdot 7H_2O$ 200 mg/l, $FeSO_4 \cdot 7H_2O$ 10 mg/l, NaCl 10 mg/l, $MnSO_4 \cdot 4H_2O$ 10 mg/l, thiamin hydrochloride 400 μg/l, nicotinic acid 400 μg/l, calcium pantothenate 400 μg/l, and p-aminobenzoic acid 100 μg/l (pH 6.5) was incorporated into each test tube having a volume of 30 ml. To the test tubes, 0.12 ml of a solution containing PQQ in different concentrations was added, and the test tubes containing the medium were sterilized at 120°C for 15 minutes.

On the other hand, cells of Gluconobacter industrius IFO 3260 were grown in the same medium mentioned above. The grown cells were washed with sterilized water, and the washed cells were then suspended in sterilized water to prepare an inoculum cell suspension.

A drop of the cell suspension was inoculated in each medium prepared as above, and culturing was carried out at a temperature of 30°C for 36 to 48 hours by shaking the test tube. Each of the cultured broths was tested for turbidity, as measured at 660 nm, to evaluate the extent of cell growth. The results are set forth in the following table and shown in Figure 1.

| Final concentration of PQQ (ng/ml) | O $D_{660}$nm |
|---|---|
| not added | 0.110 |
| 4.8 | 0.070 |
| 9.6 | 0.120 |
| 19.2 | 0.250 |
| 38.4 | 0.420 |
| 96.0 | 0.380 |

As shown by these results, at a PQQ concentration in the culture medium of up to 38.4 ng/ml, the amount of cells grown increased according to the increase of the PQQ concentration.

Example 2

5 ml of a culture medium (pH 6.4) containing 4 g/l of glycerin, 5.0 g/l of glucose, 6.0 g/l of monosodium glutamate, 10 ml/l of a solution of inorganic salt mixture having the following composition (A), and 10 ml/l of a solution of vitamin mixture having the following composition (B), was incorporated into each of two test tubes having a volume of 30 ml. PQQ was then added to one test tube to a PQQ concentration of 1 μg/ml; PQQ was not added to the other test tube. The test tubes containing the medium were sterilized at 120°C for 15 minutes.

Composition of solution A

| | |
|---|---|
| $KH_2PO_4$ | 50 g |
| $K_2HPO_4$ | 50 g |
| $MgSO_4 \cdot 7H_2O$ | 20 g |
| $FeSO_4 \cdot 7H_2O$ | 1 g |
| $MnSO_4 \cdot 4H_2O$ | 1 g |
| NaCl | 1 g |
| Distilled water | 1 ℓ |

Composition of solution B

| | |
|---|---|
| Thiamin hydrochloride | 40 mg |
| Nicotinic acid | 40 mg |
| Calcium pantothenate | 40 mg |
| p-Aminobenzoic acid | 10 mg |
| Distilled water | 1 ℓ |

On the other hand, cells of Acetobacter aceti IFO 3284 were grown in the same medium mentioned above. The grown cells were washed with sterilized water, and the washed cells were then suspended in sterilized water to prepare an inoculum cell suspension.

0.1 ml of the cell suspension was inoculated to each of the culture mediums prepared as above, and culturing was carried out at 30°C for 72 hours by shaking the test tubes on a reciprocating shaker. The turbidity of each culturing medium was measured periodically with a Klett Summerson photoelectric colorimeter to evaluate the extent of cell growth. The results are shown in Figure 2.

As seen from Figure 2, the presence of 1 µg/ml of PQQ accelerated the growth of the bacterium by about ten hours.

Example 3

100 ml of a culture medium containing 10 g/l of glucose, 10 g/l of sodium gluconate, 6 g/l of monosodium glutamate, 10 ml/l of the inorganic salt mixture solution (A) described above, and 10 ml/l of the vitamin mixture solution (B) described above was incorporated into two Sakaguchi flasks having a volume of 500 ml. PQQ was added to one of the flasks to a PQQ concentration of 1 µg/ml in the medium; PQQ was not added to the other flask. The flasks containing the medium were sterilized at 120°C for 15 minutes.

On the other hand, cells of Gluconobacter oxydans IFO 3287 were grown in the same medium mentioned above. The grown cells washed with sterilized water and the washed cells then suspended in sterilized water to prepare an inoculum cell suspension.

0.1 ml of the cell suspension was inoculated in each medium prepared as above, and culturing was carried out at 30°C for 60 hours by shaking the flasks on a reciprocating shaker. Samples were obtained periodically from each flask, and the samples were tested for turbidity as measured with a Klett Summerson photoelectric colorimeter to evaluate the extent of cell growth; for the activity of membrane-bound gluconate dehydrogenase according to a method described by Ameyama, Methods in Enzymology, 89, 20 (1982); and for the amount of 5-keto-gluconic acid produced by the culturing, according to a method described by Adachi et.al., Agricultural and Biological Chemistry, 43, 75 (1975), using 5-ketogluconate reductase.

As shown in Figure 3, the growth of the cells was accelerated by about ten hours, and times required for the production of gluconate dehydrogenase and 5-ketogluconic acid were shortened by more than ten hours.

Example 4

100 ml of a culture medium containing 4 g/l of glycerin, 6 g/l of monosodium glutamate, 10 ml/l of the inorganic salt mixture solution (A) described above, and 10 ml/l of the vitamin mixture solution (B) described above was incorporated into each of 20 Erlenmeyer flasks having a volume of 500 ml. PQQ was added to each of 10 flasks to a PQQ concentration of 0.1 μg/ml in the medium, and PQQ was not added to the other 10 flasks. All flasks containing the medium were sterilized at 120°C for 15 minutes, and after cooling, ethanol was added to each flask to an ethanol concentration of 50 ml/l, and with acetic acid to an acetic acid concentration of 10 ml/l.

On the other hand, cells of Acetobacter aceti IFO 3284 were grown in the same medium mentioned above, the grown cells were washed with sterilized water, and the washed cells were then suspended in sterilized water to prepare an inoculum cell suspension.

0.1 ml of the cell suspension was inoculated in each medium prepared as above, and static culture was carried out at 30°C for 8 days. Samples were obtained periodically from each flask. 5 ml of the sample was titrated with 0.5N NaOH, using phenolphthalein as an

indicator, to measure the acidity, which was then converted to the amount of acetic acid.

On the other hand, cells were collected from each flask, washed, and disrupted with a French pressure cell press to prepare a cell homogenate. The homogenate was then analyzed for the activities of membrane-bound alcohol dehydrogenase and aldehyde dehydrogenase according to Ameyama, Methods in Enzymology, 89, 20 (1982).

As shown in Figure 4, the addition of PQQ remarkably shortened the times required for the production of cell mass and of the above-mentioned two enzymes. Moreover, the addition of PQQ increased to some extent the maximum amounts of cell mass, and of the two enzymes.

Example 5

Using a culture medium (pH 6.5) containing 4 g/l of glycerin, 5.0 g/l of glucose, 4.0 g/l of monosodium glutamate, 10.0 g/l of sodium gluconate, 10 ml/l of the inorganic acid mixture solution (A) described above, 10 ml/l of the vitamin mixture solution (B) described above, and 0.4 mg/l of pyridoxal phosphate, and according to the procedure described in Example 2, the effect of PQQ on the growth of *Acetobacter rancens* IFO 3297 was evaluated.

As shown in Figure 5, the addition of PQQ remarkably promoted the growth of the bacterium.

Example 6

Using a culture medium (pH 6.2) containing 5 g/l of glucose, 5 g/l of $NH_4Cl$, 0.4 mg/l of thiamin hydrochloride, 0.4 mg/l of nicotinic acid, 0.4 mg/l of calcium pantothenate, 0.1 mg/l of p-aminobenzoic acid, 25 mg/l of inositol, 0.4 mg/l of pyridoxal phosphate, 0.1 mg/l of biotin, and 0.1 mg/l of riboflavin, and according to the procedure described in Example 2, the effect of PQQ on the growth of *Saccharomyces cerevisiae* (Brewing Society of Japan, *Saccharomyces sake* No. 7 yeast) was evaluated.

As shown in Figure 6, the addition of 0.1 ng/ml of

PQQ accelerated the growth of the yeast by about 15 hours.

Example 7

Using the same culture medium as described in Example 6, and according to the procedure described in Example 2, the effect of PQQ on the growth of Schizosaccharomyces pombe IFO 0346 was evaluated.

As shown in Figure 7, the addition of 0.1 ng/ml of PQQ accelerated the growth of the yeast by about five hours.

Example 8

A culture medium having the following composition was prepared.

| | | |
|---|---|---|
| Polyvinyl alcohol | 5 | g |
| $(NH_4)_2SO_4$ | 1 | g |
| $KH_2PO_4$ | 1 | g |
| $K_2HPO_4$ | 8 | g |
| $MgSO_4 \cdot 7H_2O$ | 0.2 | g |
| NaCl | 0.1 | g |
| $CaCl_2 \cdot 2H_2O$ | 0.02 | g |
| $FeSO_4 \cdot 7H_2O$ | 0.01 | g |
| $Na_2MoO_4 \cdot 2H_2O$ | 0.5 | mg |
| $Na_2WO_4 \cdot 2H_2O$ | 0.5 | mg |
| $MnSO_4$ | 0.5 | mg |
| Calcium pantothenate | 0.4 | mg |
| Inositol | 0.2 | mg |
| Niacin | 0.4 | mg |
| p-Aminobenzoic acid | 0.2 | mg |
| Pyridoxine | 0.4 | mg |
| Thiamin | 0.4 | mg |
| Biotin | 2 | μg |
| Vitamin $B_{12}$ | 0.5 | μg |
| $H_2O$ | 1000 | ml |
| pH | 7.5 | |

5 ml of the medium was incorporated into each of 7 test tubes having a volume of 30 ml, 6 test tubes were added

with 0.22, 0.56, 1.1, 2.2, 4.5, and 8.9 ng/ml of PQQ, respectively, and the remaining one test tube was used, as a control, without the addition of PQQ.

Cells of Pseudomonas sp. VM15C (Applied and Environmental Microbiology, 41, 261 (1981)) were inoculated in the test tubes, and culturing was carried out at 30°C for 120 hours by shaking the test tubes. $OD_{660}$ of the cultures were periodically determined.

As shown in Figure 8, the growth rate and the amount of cells reached at a time of 120 hours increased according to the increase of PQQ concentration.

Example 9

Ten flasks having a volume of 500 ml each containing 100 ml of an inorganic salt-sucrose medium having the following composition were prepared.

Linsmaier and Skoog Medium (E. M. Linsmaier & F. Skoog, Physiol. Plant., 18, 100 (1965))

$NH_4NO_3$, 1650 mg/L; $KNO_3$, 1900 mg/L; $CaCl_2 \cdot 2H_2O$, 440 mg/L; $MgSO_4 \cdot 7H_2O$, 370 mg/L; $KH_2PO_4$, 170 mg/L; $Na_2EDTA$, 37.3 mg/L; $FeSO_4 \cdot 7H_2O$, 27.8 mg/L; $H_3BO_3$, 6.2 mg/L; $MnSO_4 \cdot 4H_2O$, 22.3 mg/L; $ZnSO_4 \cdot 4H_2O$, 8.6 mg/L; KI, 0.83 mg/L; $Na_2MoO_4 \cdot 2H_2O$, 0.25 mg/L; $CuSO_4 \cdot 5H_2O$, 0.025 mg/L; $CoCL_2 \cdot 6H_2O$, 0.025 mg/L; Sucrose, 30 g/L; Thiamin-HCl, 0.4 mg/L; myo-Inositol, 100 mg/L; Plant growth substances: Naphthalene acetic acid $10^{-5}$ M & Benzyladenine $10^{-6}$ M pH 5.7.

Four groups of two flasks each were added with $10^{-10}$, $10^{-9}$, $10^{-8}$, and $10^{-7}$ M PQQ respectively, and the remaining group of two flasks was used, as a control, without the addition of PQQ.

To each flask, 0.55 g of living tobacco cells were inoculated, and culturing was carried out at 31°C with shaking. After 7 to 10 days, the amount of wet cells was measured.

Figure 9 represents the relationship between the concentration of PQQ expressed by -log molar concentration and the weight of wet cells per flask. The weight

of the wet cells is expressed as two values obtained from two flasks of one group and a mean value of the two values.

As seen from Figure 9, the addition of $10^{-8}$ M PQQ increased the amount of cells by about two times.

Example 10

An Eagle MEM medium having the following composition supplemented with 0.25% bovine serum was prepared.

Composition of Eagle MEM Medium

| | | |
|---|---|---|
| NaCl | 6.8 | g |
| KCl | 400 | mg |
| $Na_2HPO_4$ | 115 | mg |
| $MgSO_4$ | 93.5 | mg |
| $CaCl_2$ | 200 | mg |
| Glucose | 1000 | mg |
| L-Arginine hydrochloride | 126 | mg |
| L-Cysteine hydrochloride monohydrate | 31.4 | mg |
| L-Tyrosine | 36 | mg |
| L-Histidine hydrochloride monohydrate | 42 | mg |
| L-Isoleucine | 52 | mg |
| L-Leucine | 52 | mg |
| L-Lysine hydrochloride | 75 | mg |
| L-Methionine | 15 | mg |
| L-Phenylalanine | 32 | mg |
| L-Threonine | 48 | mg |
| L-Tryptophan | 10 | mg |
| L-Valine | 46 | mg |
| Succinic acid | 75 | mg |
| Sodium succinate | 100 | mg |

| | | |
|---|---|---|
| Choline bitartrate | 1.8 | mg |
| Folic acid | 1 | mg |
| iso-Inositol | 2 | mg |
| Nicotinamide | 1 | mg |
| Calcium pantothenate | 1 | mg |
| Pyridoxal hydrochloride | 1 | mg |
| Riboflavin | 0.1 | mg |
| Thiamin hydrochloride | 1 | mg |
| Biotin | 0.02 | mg |
| Kanamycin | 60 | mg |
| Phenol red | 6 | mg |
| L-Glutamine | 292 | mg |

10% $NaHCO_3$ solution 12.5 to 22 ml (pH 7.1 to 7.4).

Moreover, PQQ-containing Eagle MEM media containing 0.033, 0.33, and 3.3 ng/ml of PQQ, respectively, were prepared.

Culturing was carried out in a $CO_2$- incubator at 37°C in an atmosphere of 5% $CO_2$/95% air. 2 ml of a suspension of HeLa cell in the Eagle MEM medium was incorporated into each of eight petri dishes having a diameter of 3.5 cm. After one day, the supernatants in two petri dishes were exchanged by fresh Eagle MEM medium; supernatants in another two petri dishes were exchanged by Eagle MEM medium supplemented with 0.033 mg/ml of PQQ; and in the same way, supernatants in the other four petri dishes were exchanged by Eagle MEM media supplemented with 0.33, and 3.3 ng/ml of PQQ, respectively.

Culturing was continued for 48 hours, and samples were obtained at 24 hours and at 48 hours. The number of cells in the samples was counted, and two values from

two cultures containing the same amount of PQQ were averaged.

The results are set forth in the following table.

| PQQ concentration (ng/ml) | Culture time (hr) 0 | 24 | 48 |
|---|---|---|---|
| 0 | 5 | 6 | 7 |
| 0.033 | 5 | 6 | 7.2 |
| 0.33 | 5 | 6.4 | 10 |
| 3.3 | 5 | 7 | 70 |

As seen from the table, the number of HeLa cells increased according to an increase of PQQ concentration.

Example 11

A Nissui Eagle MEM medium (commercially available from Nissui Seiyaku Co.) supplemented with 0.25% lacto-albumin hydrolyzate and 0.292 g/l monosodium glutamate, and the same medium further supplemented with $1.3 \times 10^{-6}$ M (0.43 μg/ml) PQQ, were prepared.

Vero cells (African green monkey established cells) were inoculated in each of the above-mentioned two media at a concentration of $7 \times 10^4$ cells/ml, and cultured at 30°C. The number of cells was counted periodically. The results are shown in Figure 10.

As seen from Figure 10, $1.3 \times 10^{-6}$ M PQQ promoted the cell growth.

The results obtained from the Examples are summarized in the following table.

| Exp. No. | Cell | Effect shown in Exp. | Utility at present |
|---|---|---|---|
| 1 | Gluconobacter industrius IFO 3260 | Promoted cell growth | Production of gluconic acid, 5-ketogluconic acid and dihydroxyacetone |
| 2 | Acetobacter aceti IFO 3284 | Promoted cell growth | Production of acetic acid |
| 3 | Gluconobacter oxydans IFO 3287 | (1) Promoted cell growth<br>(2) Promoted production of gluconate dehydrogenase<br>(3) Promoted production of 5-ketogluconic acid | Production of 5-ketogluconic acid, dihydroxyacetone and L-sorbose |
| 4 | Acetobacter aceti IFO 3284 | (1) Promoted production of alcohol dehydrogenase and aldehyde dehydrogenase<br>(2) Promoted production of acetic acid | Production of acetic acid |
| 5 | Acetobacter rancens IFO 3297 | Promoted cell growth | Production of acetic acid |

| Exp. No. | Cell | Effect shown in Exp. | Utility at present |
|---|---|---|---|
| 6 | Saccharomyces cerevisiae (Brewing Society of Japan, Saccharomyces sake No. / yeast) | Promoted cell growth | Production of Ethanol and vaccine |
| 7 | Schizosaccharomyces pombe (IFO 0340) | Promoted cell growth | Production of Ethanol |
| 8 | Pseudomonas sp. VM15C | Promoted cell growth | Assimilation of polyvinyl alcohol (expected to be used for purification of waste) |
| 9 | Tobacco cells | Promoted cell growth | |
| 10 | Hela cells | Promoted cell growth | |
| 11 | Vero cells (African green monkey established cell) | Promoted cell growth | |

As seen from the above-described Tables, Figures, and Examples, a growth promoting agent and a process for the promotion of cell growth according to the present invention effectively promotes or accelerates the growth of microorganisms, plant and animal cells, which in turn promotes the production of an aimed product. Namely, the times required to reach the maximum yield amount of cells mass and of the aimed products other than cells are shortened, and in some cases, the maximum yield amount obtained is elevated. Moreover, as PQQ, an active ingredient used for the present invention, is a stable, low-molecular weight compound, and its effective addition amount is very low in comparison with conventional growth promoting agents derived from natural products or extracts thereof, isolation and purification of an aimed product after culturing are not adversely affected by the addition of PQQ.

Therefore, the agent and process of the present invention are extremely useful and valuable from a practical, industrial, and economical point of view.

CLAIMS

1. A cell growth promoting agent for microorganism, plant and animal cells, comprising 4,5-dihydro-4,5-dioxo-1H-pyrrolo[2,3-f]quinoline-2,7,9-tricarboxylic acid (PQQ) or salt thereof.

2. A cell growth promoting agent according to claim 1, wherein the microorganism cells are cells of bacterium selected from the group consisting of genera Gluconobacter and Acetobacter.

3. A cell growth promoting agent according to claim 1, wherein the microorganism cells are cells of yeast selected from the group consisting of Saccharomyces and Schizosaccharomyces.

4. A cell growth promoting agent according to claim 1, wherein the plant cells are tobacco cells.

5. A cell growth promoting agent according to claim 1, wherein the animal cells are selected from the group consisting of HeLa cells and Vero cells.

6. A process for promoting growth of microorganism, plant or animal cells, comprising culturing the cells in a culture medium containing an effective amount of 4,5-dihydro-4,5-dioxo-1H-pyrrolo[2,3-f]quinoline-2,7,9-tricarboxylic acid (PQQ) or salt thereof.

7. A process according to claim 6, wherein further the production of an aimed product other than cells is promoted.

8. A process according to claim 7, wherein a time required for production of the aimed product is shortened.

9. A process according to claim 7, wherein an amount of the aimed product produced during the culturing is increased.

10. A process according to claim 6, wherein the culture medium is a conventional medium, and PQQ is added to the conventional medium in the total amount of 0.01 ng to 50 μg per ml of the medium.

11. A process according to claim 6, wherein the

microorganism cells are cells of bacterium selected from the group consisting of genera Gluconobacter and Acetobacter.

12. A process according to claim 6, wherein the microorganism cells are cells of yeast selected from the group consisting of Saccharomyces and Schizosaccharomyces.

13. A process according to claim 6, wherein the plant cells are tobacco cells.

14. A process according to claim 6, wherein the animal cells are selected from the group consisting of HeLa cells and Vero cells.

0174155

Fig. 1

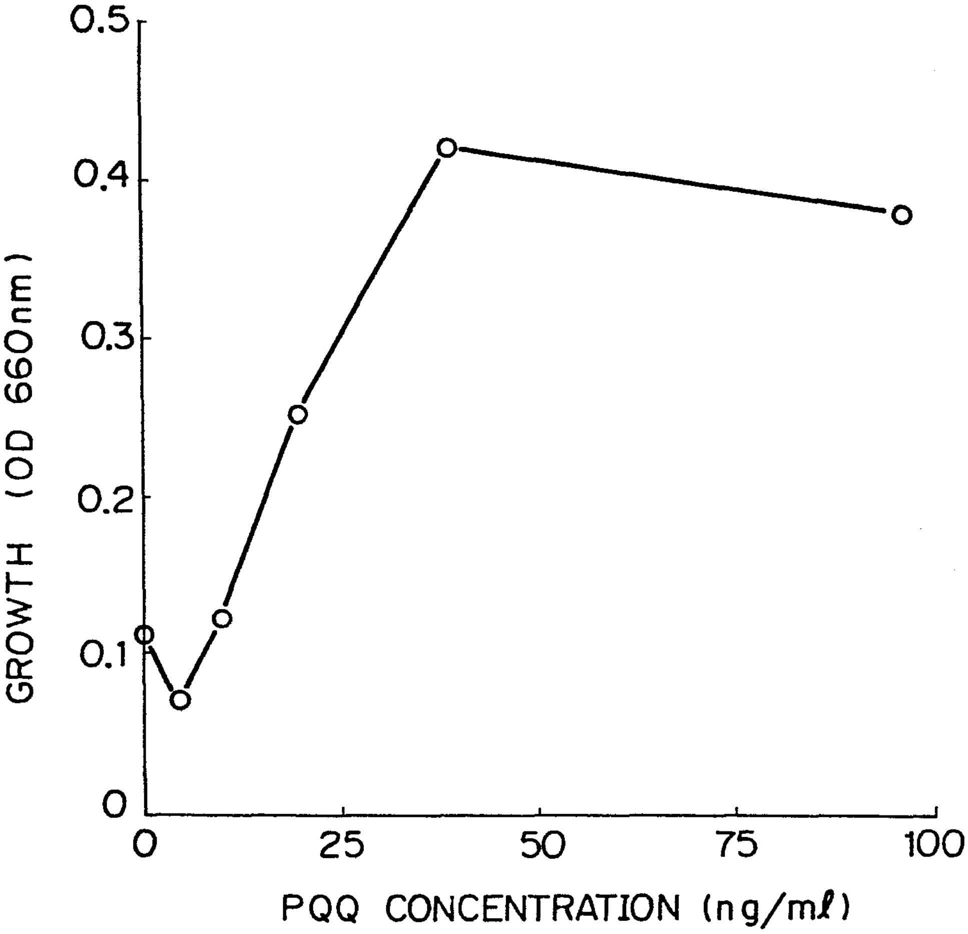

*Fig. 2*

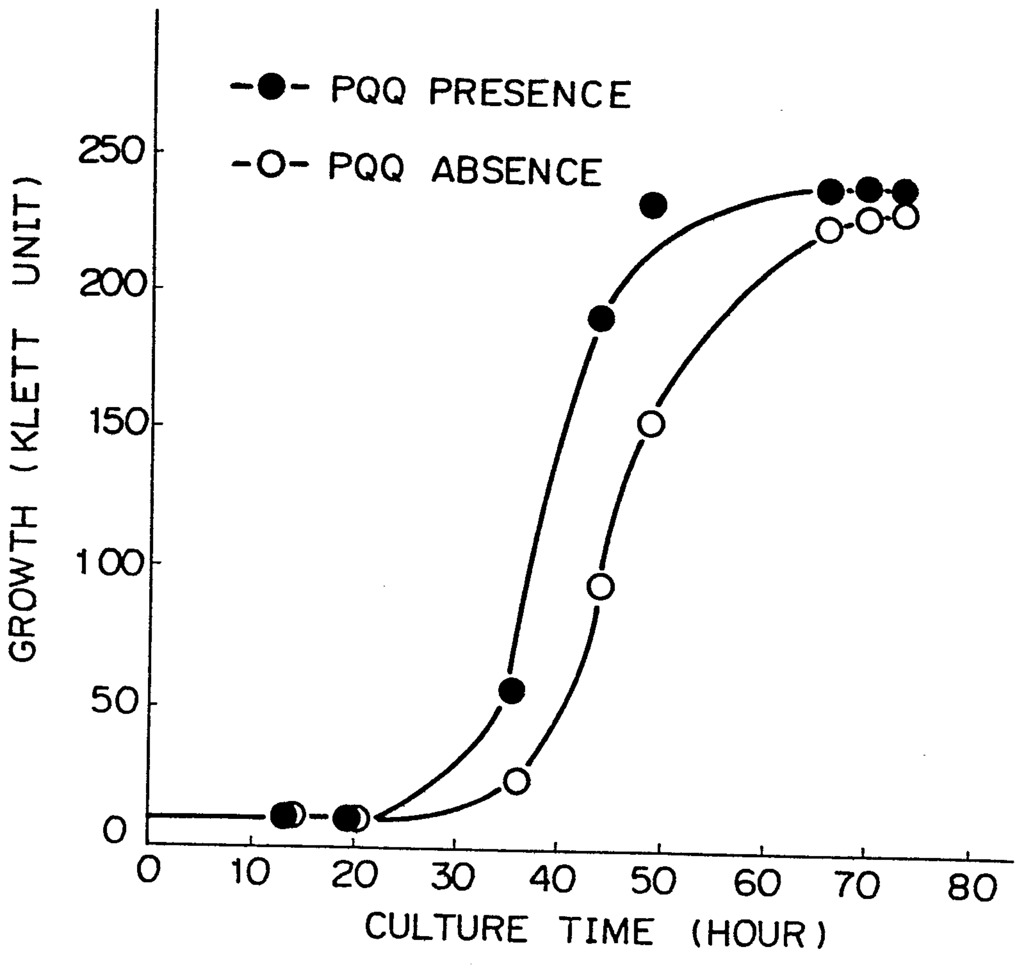

-●- PQQ PRESENCE
-○- PQQ ABSENCE
GROWTH (KLETT UNIT)
250
200
150
100
50
0
0
10
20
30
40
50
60
70
80
CULTURE TIME (HOUR)

# Fig. 3

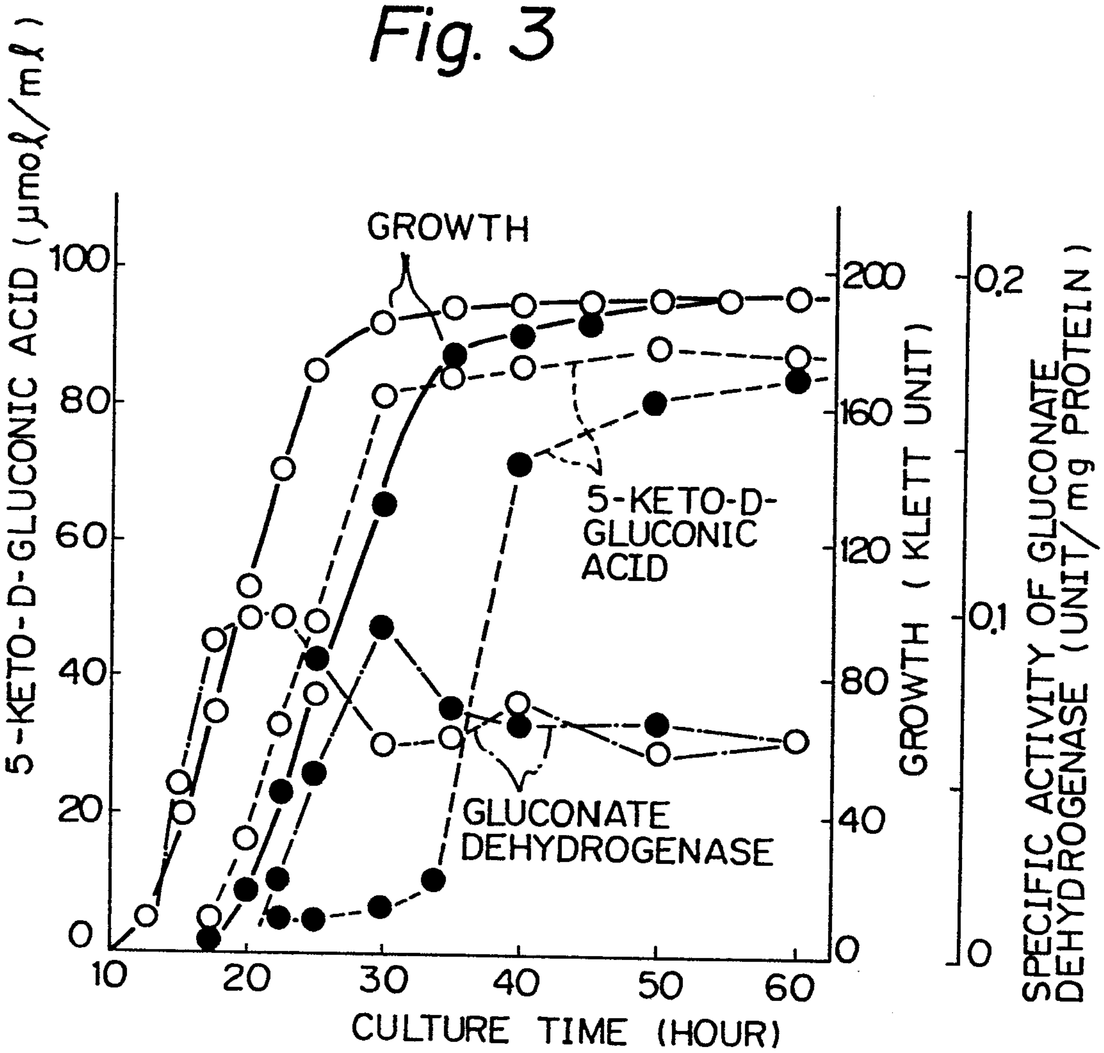


PQQ ABSENCE
- —●— GROWTH
- ---●--- 5-KETO-D-GLUCONIC ACID
- -·-●-·- SPECIFIC ACTIVITY OF MEMBRANE-BOUND GLUCONATE DEHYDROGENASE

PQQ 1μg/mℓ
- —○—
- ---○---
- -·-○-·-

SAME AS ABOVE

*Fig. 4*

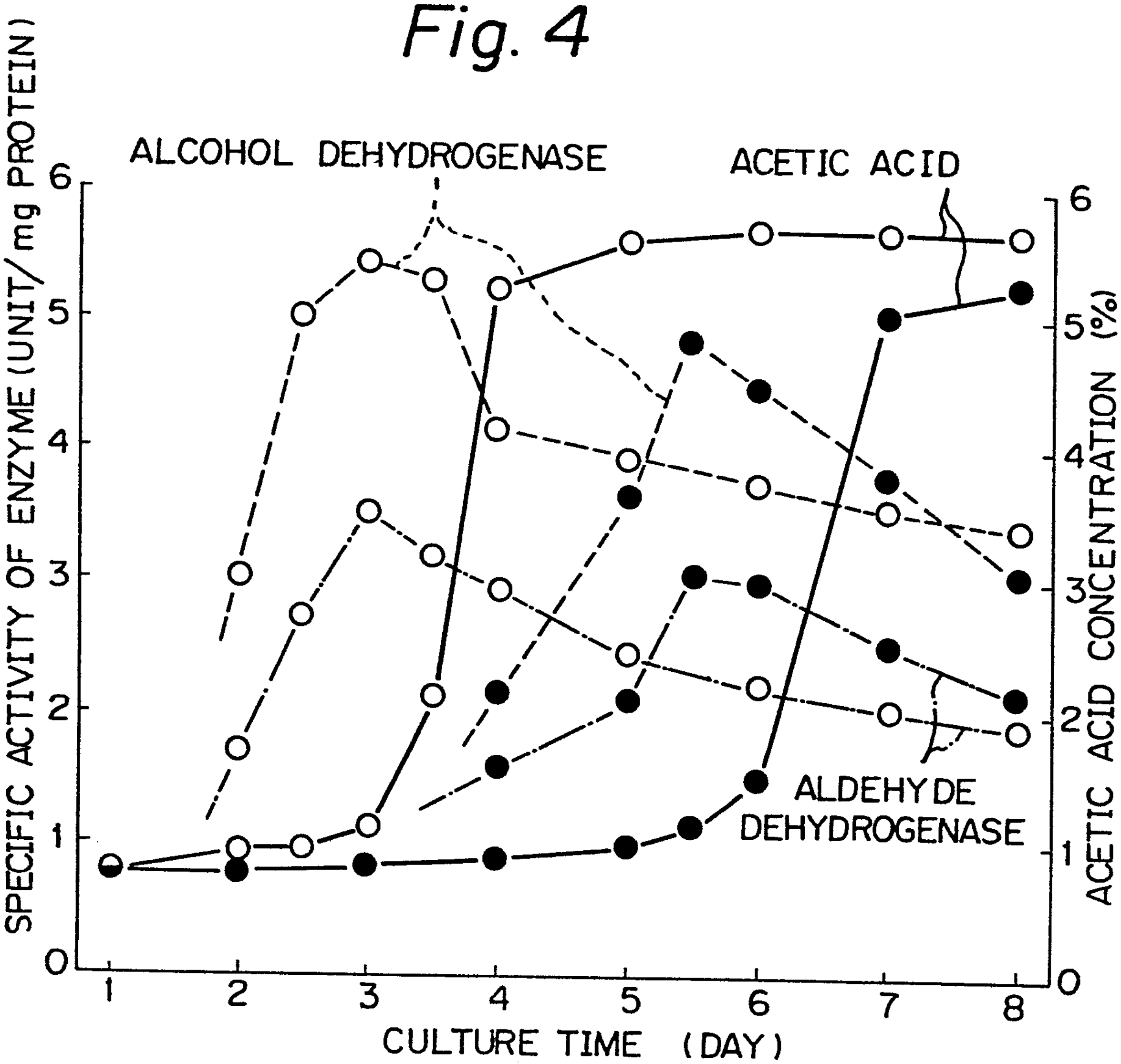


PQQ ABSENCE
- —●— ACETIC ACID
- ---●--- MEMBRANE-BOUND ALCOHOL DEHYDROGENASE
- —·—●—·— MEMBRANE-BOUND ALDEHYDE DEHYDROGENASE

0.1μg/ml PQQ
- —○—
- ---○---
- —·—○—·—

SAME AS ABOVE

Fig. 5

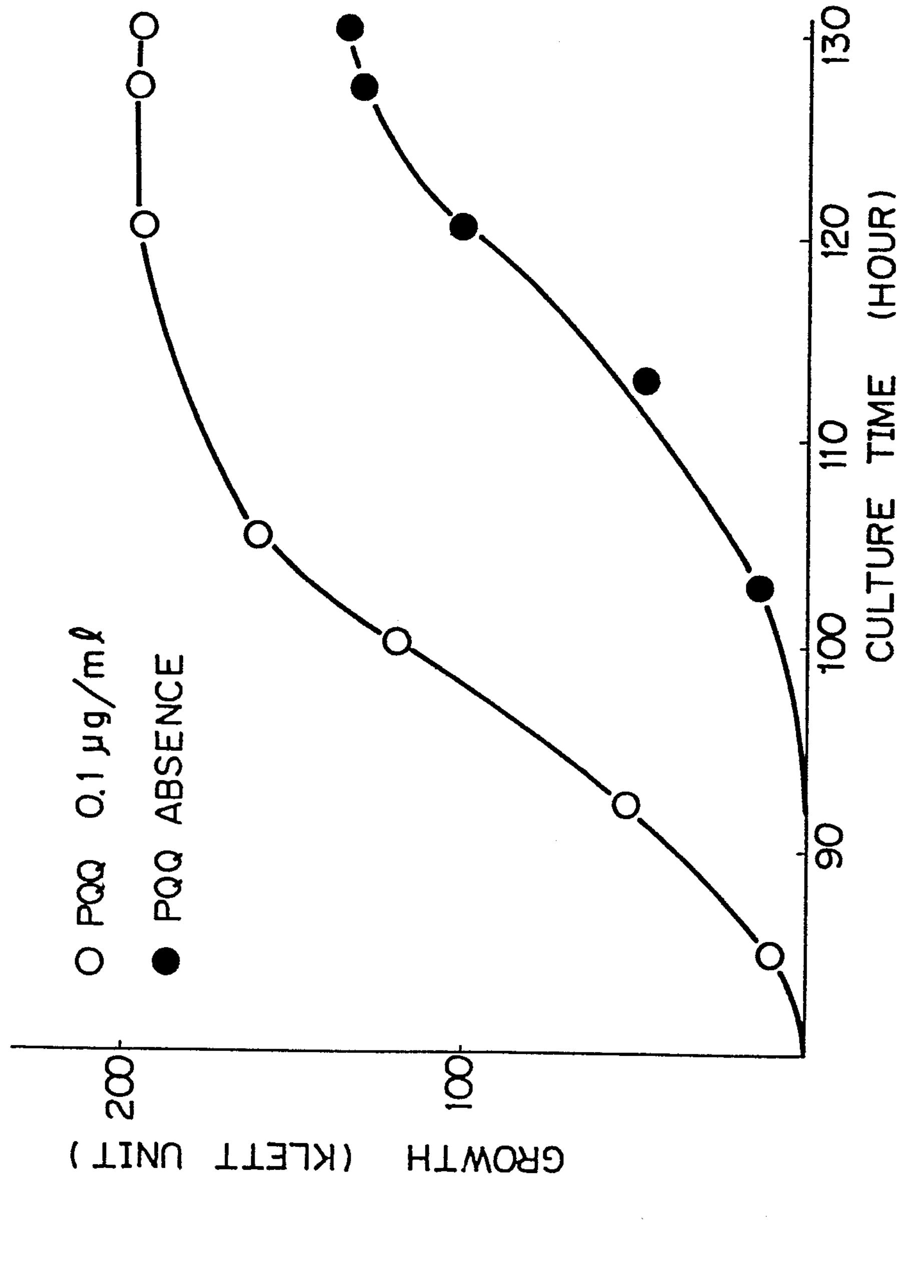
○ PQQ 0.1 μg/mℓ
● PQQ ABSENCE
GROWTH (KLETT UNIT)
200
100
90
100
110
120
130
CULTURE TIME (HOUR)

# Fig. 6

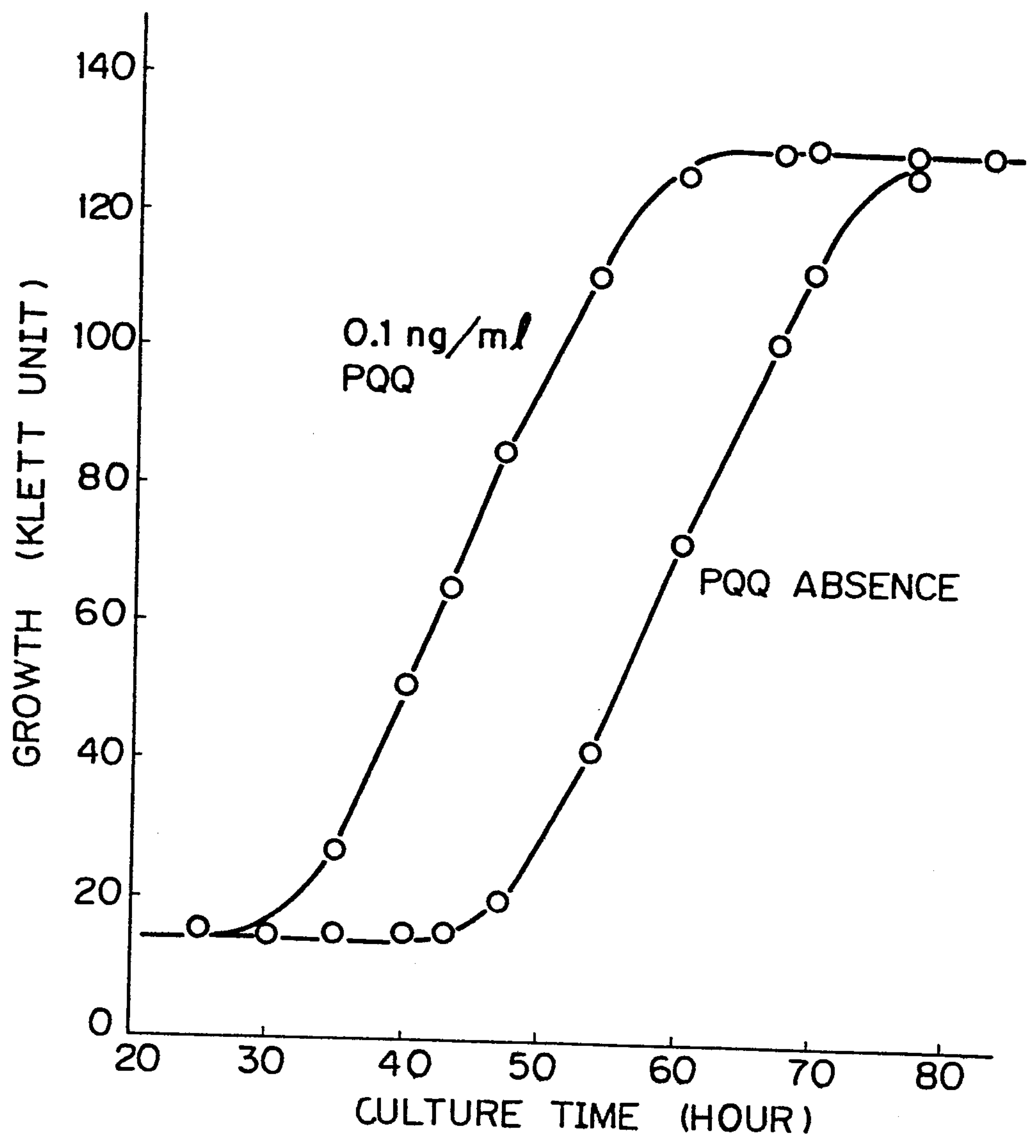
140
120
100
80
60
40
20
0
GROWTH (KLETT UNIT)
0.1 ng/ml
PQQ
PQQ ABSENCE
20
30
40
50
60
70
80
CULTURE TIME (HOUR)

*Fig. 7*

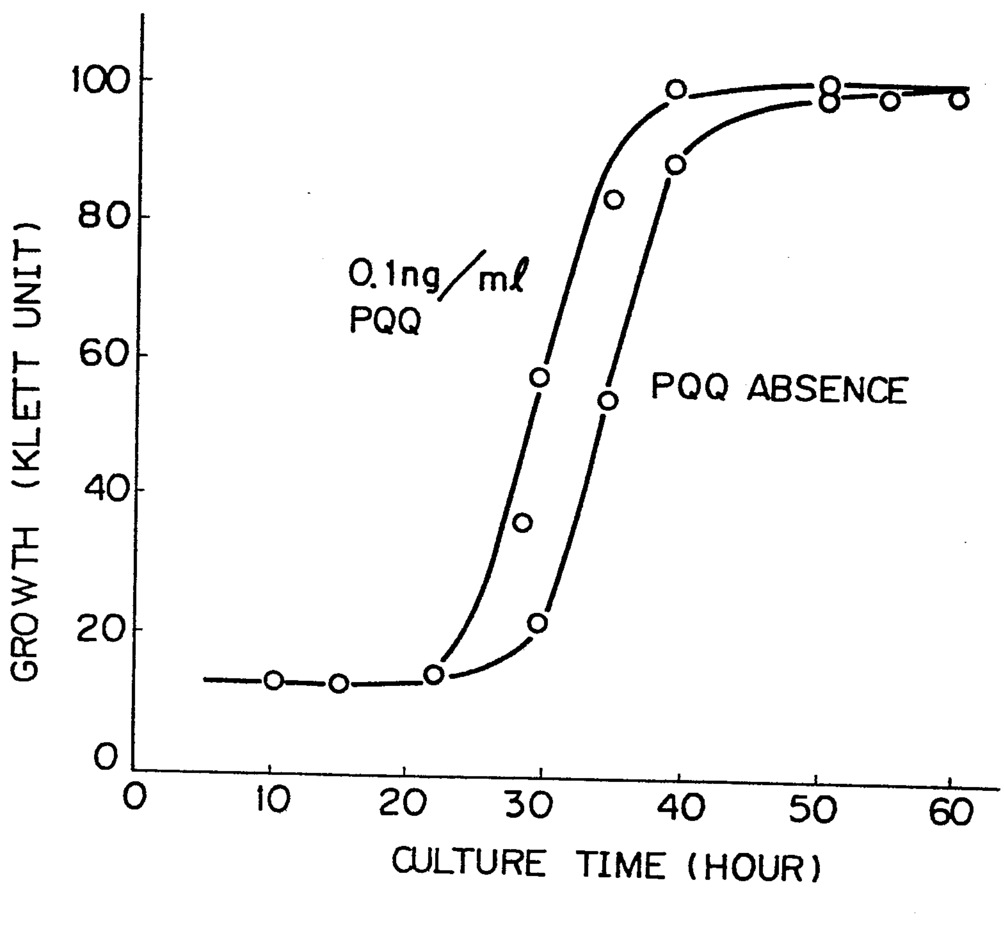
GROWTH (KLETT UNIT)
100
80
60
40
20
0
0
10
20
30
40
50
60
CULTURE TIME (HOUR)
0.1ng/ml
PQQ
PQQ ABSENCE

0174155

Fig. 8

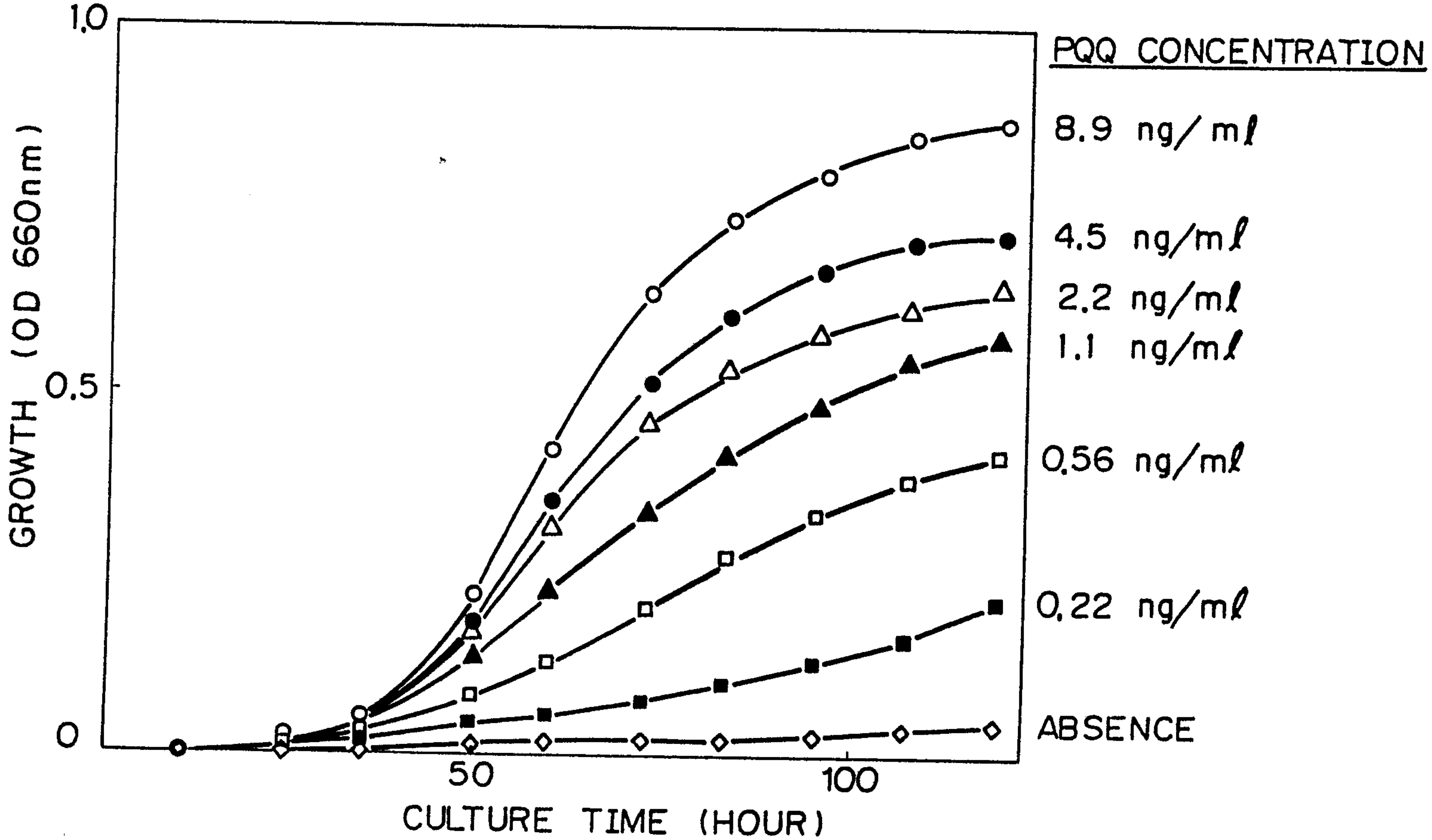

Fig. 9

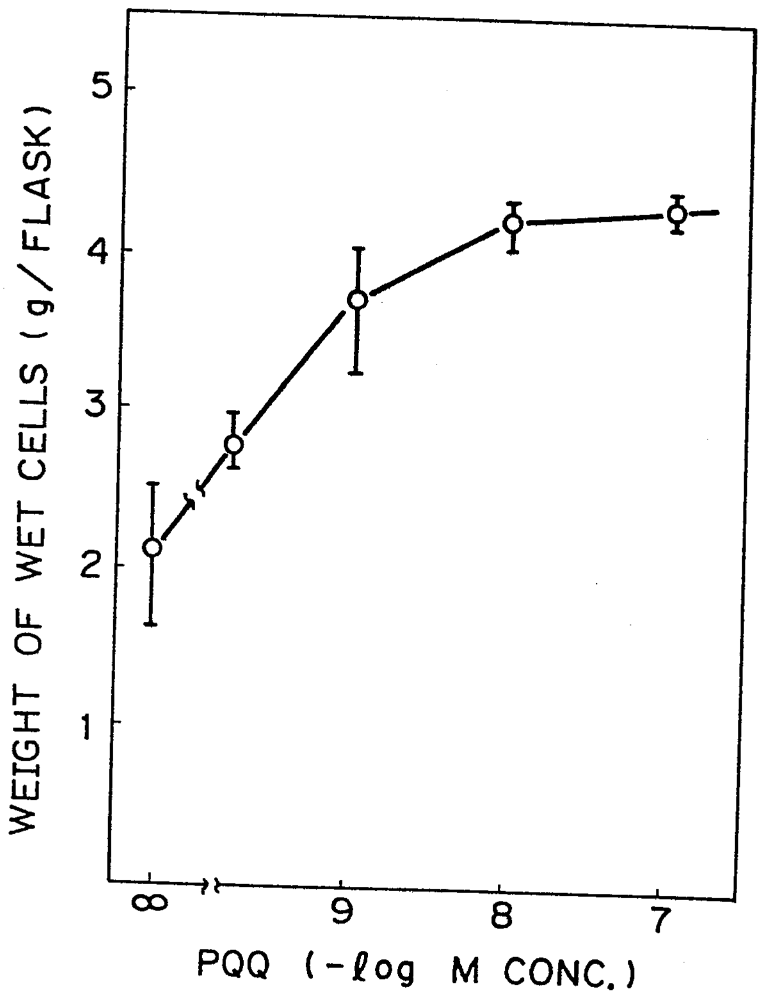

0174155

Fig. 10

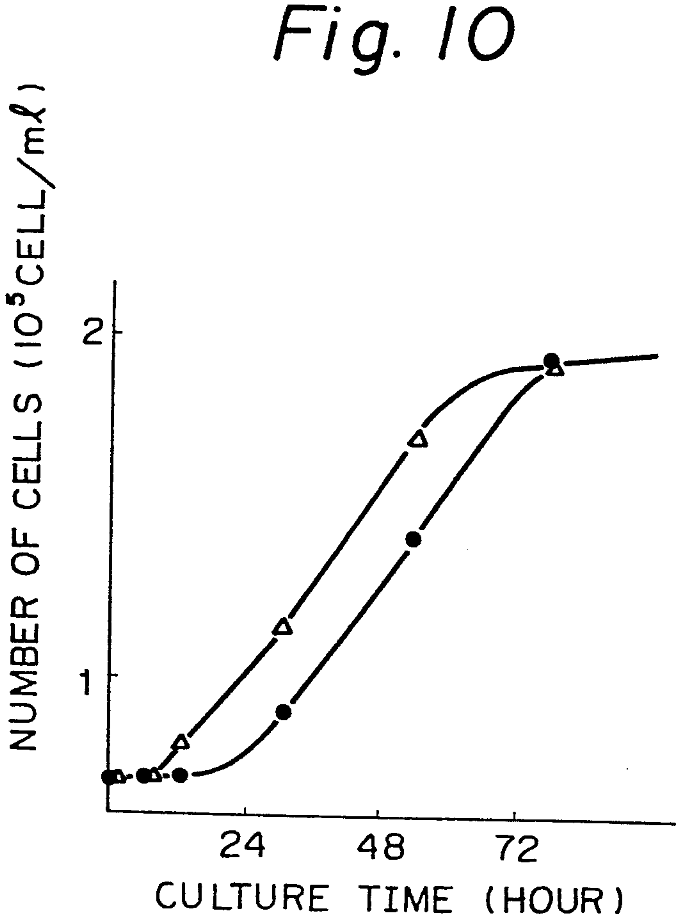


● : PQQ ABSENCE

△ : PQQ $1.3 \times 10^{-6}$ M